# EUROPÄISCHE PATENTSCHRIFT

(11) **EP 0 587 047 B1**
(45) Veröffentlichungstag und Bekanntmachung des Hinweises auf die Patenterteilung: **12.12.2001**
(21) Anmeldenummer: 93114056.0
(22) Anmeldetag: 02.09.1993
(51) Int. Cl.: A61K 47/32, A61K 31/57, A61P 15/12

(54) **Pharmazeutische Zubereitung auf Gestagen-Basis**
Gestagenes containing pharmaceutical composition
Composition pharmaceutique contenant des gestagènes

(30) Priorität: 07.09.1992 DE 4229820
(43) Veröffentlichungstag der Anmeldung: 16.03.1994
(73) Patentinhaber: Jenapharm GmbH & Co. KG, 07745 Jena (DE)
(72) Erfinder: Oettel, Michael, Prof., D-07743 Jena (DE); Osterwald, Hermann, Dr., D-07743 Jena (DE); Dittgen, Michael, Prof., D-99510 Apolda (DE)

(56) Entgegenhaltungen:
- WO-A-87/04342
- WO-A-90/10425
- PATENT ABSTRACTS OF JAPAN vol. 13, no. 431 (C-640), 26.September 1989 & JP-A-01 168619 (TAKADE SEIYAKU KK), 4.Juli 1989,

## Beschreibung

Die Erfindung betrifft eine pharmazeutische Zubereitung auf Gestagen-Basis zum Einsatz in der sogenannten Hormone-Replacement-Therapie, d. h. zur Behandlung klimakterischer Beschwerden, des Postmenopausen-Syndroms und der Osteoporose.

Seit langem ist bekannt, daß mit weiblichen Sexualsteroiden, den Östrogenen, eine wirksame Therapie klimakterischer Beschwerden oder des Postmenopausen-Syndroms möglich ist. Darüber hinaus verhindern Östrogene den Skelett-Abbau, d. h. die Ausbildung einer Osteoporose nach Fortfall der körpereigenen Östrogensekretion in der Postmenopause.

Bei oraler Einnahme endometriotroper Östrogene, wie Ethinylestradiol, Estradiol und seinen Estern (z. B. dem Valerat) sowie von konjugierten Östrogenen (einschließlich Estronsulfat) oder bei parenteraler Gabe langwirksamer Estradiolderivate wird gegenwärtig zu Recht der zyklische Einsatz von Gestagenen in der zweiten Behandlungshälfte (sequentiell) über 10 bis 14 Tage, analog dem natürlichen Menstruationszyklus gefordert. Dieser Forderung nachzukommen ist auch Anliegen der Erfindung. Sofern der Uterus noch vorhanden ist und ein normaler Zyklus mit 28tägigen Blutungen angestrebt wird, bewirkt der sequentielle Gestagenzusatz eine sekretorische Transformation des Endometriums mit einer regelrechten menstruellen Abstoßung der Schleimhaut. Mit dieser Empfehlung wird das Auftreten einer Endometriumshyperplasie mit azyklischen oder Dauerblutungen verhütet. Nach langzeitiger monophasischer Östrogensubstitution ohne Gestagenzusatz kann es zur Entstehung eines Endometrium-(Corpus)-Carcinoms kommen. Das Risiko der Entstehung eines solchen Carcinoms ist nach US-amerikanischen Untersuchungen auf das 3- bis 12- fache erhöht, wenn nur Östrogene ohne sequentiellen Gestagenzusatz verabfolgt werden.

Alle bisher bekannten Untersuchungen zeigen, daß ein sequentieller Gestagenzusatz über 10 bis 14 Tage die Entwicklung einer glandulär-zystischen Hyperplasie fast immer vollständig verhindert. Die Entstehung eines Corpus-Carcinoms wird gegenüber unbehandelten Vergleichsgruppen um bis zu 78 % reduziert. Der Gestagenzusatz stellt also für das Endometrium eine echte, hochwirksame Carcinomprophylaxe dar.

Bei Frauen in der Postmenopause, die eine Langzeitbehandlung ohne Beibehaltung des Menstruationszyklus erhalten, wird eine kontinuierliche Substitution mit einem Östrogen-Gestagen-Präparat empfohlen. Bei dieser Therapie wird die Gestagendosis so eingestellt, daß nach 4 bis 5 Monaten eine Endometriumsatrophie und damit eine Amenorrhoe eintritt. Es wird angenommen, daß auch diese Art der Substitution das Risiko eines Endometriumcarcinoms vermindert (C. LAURITZEN, Gutachten zur Frage: Ist bei Östrogenmedikation die Zugabe eines Gestagens erforderlich? 1992).

Als Gestagene werden im Prinzip alle oral wirksamen Gestagene verwendet, die auch in verschiedene Kontrazeptiva Eingang gefunden haben, wie z. B. Chlormadinonacetat, Norgestimat, Lynestrenol, Levonorgestrel, Cyproteronacetat, Desogestrel, Gestoden und Norethisteron bzw. dessen Acetat.

In letzter Zeit wurde nachgewiesen, daß auch das natürliche Progesteron zur Prävention einer Osteoporose geeignet ist (J. R. LEE, Medical Hypotheses 35:316-318, 1992).

Während sich für die Behandlung des Postmenopausen-Syndroms mit Östrogenen, wie mit dem 17β-Estradiol, transdermale therapeutische Systeme (TTS) bereits auf dem Markt durchgesetzt haben, gelang es bisher nicht in befriedigender Weise, auch das notwendige Gestagen in Form eines TTS zu applizieren. Es ist lediglich bekannt, Norethisteronacetat transdermal zu verabreichen, wobei in der zweiten Zyklushälfte täglich über 4 Tage 0.25 mg Norethisteronacetat (NETA) aus einem TTS freigesetzt werden. Da das NETA neben dem Estradiol zu applizieren ist, muß aller 4 Tage das TTS, das das NETA neben dem Estradiol enthält, erneuert werden, wobei die Freisetzung des Estradiol aus dem TTS durch die simultane Applikation des NETA verzögert wird [SCHENKEL, Plenarvortrag anläßlich des 7th Congress of the European Association of Gynecologists (EAGO), 28.06.bis 01.07.1992 Helsinki].

Nachteilig bei dieser Art der Verabreichung ist weiterhin, daß das NETA antiöstrogene Wirkungen aufweist, die den Effekt der Hormone Replacement-Therapie (HRT) mit simultaner Östrogengabe reduziert. Ferner sind mit der Anwendung von NETA androgene Effekte verbunden, die sich insbesondere im Auftreten von ohnehin in der Postmenopause auftretenden Virilierungserscheinungen äußern. Die Transformationsdosis liegt für NETA mit 50 mg/Zyklus relativ hoch, d. h. es muß im Vergleich zu anderen Gestagenen - deren transdermale Applikation bisher nicht gelungen ist - höher dosiert werden, um die notwendige Transformation des Endometriums zu bewirken (KUHL/TAUBERT: Das Klimakterium, Georg Thieme Verlag, Stuttgart, 1987; siehe nachfolgende Tabelle).

| Gestagen | Tranformationsdosis (mg/Zyklus) |
|---|---|
| Gestoden | 3 |
| Desogestrel | 2 |
| Levonorgestrel | 6 |
| Norgestimat | 7 |
| Norethisteronacetat | 50 |

Aufgabe der vorliegenden Erfindung ist es nun, durch neuartige pharmazeutische Zubereitungen die Verabreichung des Gestagens, insbesondere eines von antiöstrogenen und androgenen Wirkungen freien Gestagens, zu ermöglichen. Diese Zubereitungen sollen durch einen engen Kontakt zum Resorptionsorgan (Haut, Schleimhaut) die verlustfreie Resorption des Gestagens ermöglichen.

Die Aufgabe wird erfindungsgemäß durch eine pharmazeutische Zusammensetzung auf Gestagen-Basis gelöst, die zu einem Anteil aus einem Carboxyvinylpolymeren oder dessen Neutralisationsprodukt besteht, in das das Gestagen eingebettet ist und die pharmazeutische Zubereitung aus Gelen und/oder sich im Kontakt mit dem Organismus in Gele umwandelnde überzogenen Arzneiformen, Salben, transdermalen therapeutischen Systemen oder anderen Zubereitungen besteht.

Eine vorteilhafte Ausgestaltung der Erfindung ist, daß der Anteil des Carboxyvinylpolymeren zwischen 0,5 und 50 % beträgt und daß das Carboxyvinylpolymere aus Copolymerisaten aus einem oder mehreren Acryl- und/ oder Methacrylestern mit Acryl-, Methacryl-, Itacon-, Croton-, Malein- oder Fumarsäure besteht.

Eine weitere vorteilhafte Ausgestaltung der pharmazeutischen Zubereitung ist, daß die Copolymerisate einen Säureanteil zwischen 5 und 25 % aufweisen.

Ferner ist vorteilhaft, daß die Neutralisationsprodukte der Carboxyvinylpolymere mit Natronlauge, mit Kalilauge, Stickstoffbasen oder anderen basischen Substanzen ganz oder teilweise neutalisierte Carboxyvinylpolymere darstellen.

Auch stellt eine vorteilhafte Ausgestaltung der Erfindung dar, daß das Gestagen ein Steroid aus der Gruppe Dienogest, Chlormadinonacetat, Norgestimat, Lynestrenol, Levonorgestrel, Cyproteronacetat, Desogestrel, Gestoden sowie Norethisteronacetat darstellt.

Vorteilhaft ist auch die Ausgestaltung, daß das Gel zuerst als Tablette, Dragee oder Kapsel vorliegt, die sich im Organismus im Ergebnis des Zerfalls in ein Gel umwandelt.

Die folgenden Beispiele erläutern die Erfindung in nicht beschränkender Weise.

### Ausführungsbeispiele

### Beispiel 1

### Bioadhäsive Kapsel mit Desogestrel

| | |
|---|---|
| Arzneistoffe | 1 Kapsel enthält: Desogestrel 0,2 mg. |
| Hilfsstoffe | Polyacrylatdispersion (Eudragit^{R} E 30 D), Lactose, Kartoffelstärke, Natriumcarboxymethylstärke, Magnesiumstearat, Gelatine, Ethanol, Wasser, Farbstoff E 123. |

### Herstellung:

Desogestrel wird in mikronisierter Form der Mischung aus Lactose, Kartoffelstärke und Natriumcarboxymethylstärke zugesetzt. Auf diese Mischung wird in der Wirbelschicht die gegebenenfalls verdünnte Polyacrylatdispersion aufgesprüht.

Im gleichen Arbeitsgang wird aus dieser Mischung ein Granulat geformt, das nach Zusatz des Gleitmittels Magnesiumstearat in bekannter Weise in Hartgelatinesteckkapseln eingefüllt wird.

Die Bioadhäsion des in Darmflüssigkeit zerfallenen Arzneimittels (Schleim) beträgt je nach Flüssigkeitsangebot zwischen 80 und 480 Pa. Der Schleim haftet an der Darmschleimhaut. Der enthaltene Wirkstoff kann durch die Schleimschicht diffundieren, in die Darmschleimhaut eindringen und systemisch wirksam werden.

### Beispiel 2

### Bioadhäsive Vaginaltablette mit Desogestrel

| | |
|---|---|
| Arzneistoffe | 1 Vaginaltablette enthält: Desogestrel 0.12 mg. |
| Hilfsstoffe | Polyacrylatdispersion (Acrylsäure-Acrylsäureethylester-Copolymerisat 20/80), Lactose, Kartoffelstärke, Natriumcarboxymethylstärke, Magnesiumstearat. |

### Herstellung:

Ein nach Beispiel 1, jedoch anstelle von Eudragit^{R} mit einer 40 %igen Dispersion eines Acrylsäure-Acrylsäureethylester-Copolymerisates 20/80 hergestelltes Granulat wird, wie allgemein üblich, zu oblongen Tabletten mit einem großen Durchmesser von 20 mm verpreßt. Die Bioadhäsion des im Vaginalschleim zerfallenden Arzneimittels (Schleim) beträgt je nach Flüssigkeitsangebot zwischen 100 und 500 Pa. Der Schleim haftet bis zu 48 h an der Vaginalschleimhaut. Der enthaltene Wirkstoff kann durch die Schleimschicht diffundieren, in tiefere Schichten eindringen und systemisch wirksam werden.

### Beispiel 3

### Transdermales therapeutisches System (TTS)

| | |
|---|---|
| Arzneistoff: Desogestrel | |
| Herstellung: Das TTS wird aus zwei Ansätzen hergestellt: | |
| Ansatz 1 enthält mg/TTS | |
| Desogestrel | 0.090 |
| Ethanol | 7.000 |
| Wasser | 5.000 |
| Ansatz 2 enthält mg/TTS | |
| Eudragit L 100 | 1.410 |
| Eudragit S 100 | 1.410 |
| Farbstoff E 123 | 0.020 |
| Glycerol | 0.500 |
| Wasser | 14.000 |

Ansatz 1 und Ansatz 2 werden gemischt. Die Mischung in geeigneter Weise z.B. im Gießverfahren oder durch Rakelauftrag dünn ausgestrichen und zum Film getrocknet. Auf den getrockneten Film wird in dünner Schicht eine bioadhäsive Haftschicht (z. B. aus dem erwähnten Acrylsäure-Acrylsäureethylester-Copolymerisat 20/80) aufgebracht, so daß ein Laminat entsteht. Danach wird das Laminat erneut getrocknet.

Es ist auch möglich, das Laminat im sogenannten "Transferverfahren" durch Kalandrierung des Filmes, der die Arzneistoffe enthält, mit einem geeigneten Haftfilm herzustellen. Die Haftschicht kann vor der Anwendung des Transdermalpflasters in bekannter Weise mit einer Schutzfolie bedeckt sein.

Das Transdermalpflaster haftet auf der menschlichen Haut, auch wenn diese leicht behaart ist . Die Haftkraft beträgt zwischen 100 und 500 Pa. Der enthaltene Wirkstoff kann durch den Haftfilm diffundieren, in die Haut eindringen und systemisch wirksam werden.

### Beispiel 4

Bei Anwendung der erfindungsgemäßen Zubereitung in Form eines in Anlehnung an Beispiel 3 hergestellten Dienogest-TTS in Kombination mit Estradiol als Estradiol-Patch oder als per oral applizierbare Zubereitung von mikronisiertem Estradiol wurden folgende Werte ermittelt:
Freisetzungsrate Dienogest: 0.125 mg/Tag
Peak der Freisetzung: 24 h nach Aufkleben des Pflasters Serumkonz. (radioimmunologisch bestimmt): 5 bis 15 ng/ml
Freisetzungrate (Estradiol-Patch): 0.05 mg/Tag
Peak der Freisetzung: 48 h
Serumkonzentration: 40 bis 50 pg/ml

Ein Patch, der täglich 0.250 mg Dienogest freisetzt, ist für die alleinige Behandlung im Rahmen der HRT, d. h. ohne Östrogenkomponente, geeignet. Die Serumkonzentrationen liegen dann zwischen 10 und 20 ng Dienogest/ml.

Im Gegensatz zu der aus dem Stand der Technik bekannten verzögerten Estradiol-Freisetzung bei Estradiol-Gestagen-Kombinationen als TTS mit NETA als Gestagen-Komponente wurde bei Applikation der erfindungsgemäßen Zubereitung im Vergleich der Freisetzungsprofile von getrennt applizierten Estradiol- und Dienogest-Patches mit der Kombination eines Dienogest/ Estradiol-Patches keine negative Beeinflussung der Freisetzung bzw. Hautpenetration der Wirkstoffe festgestellt.

Die direkte Osteoporose-Wirksamkeit von Dienogest wurde an einem experimentellen Osteoporose-Modell mit ovarektomierten weiblichen Ratten geprüft. Nach Ovarektomie stiegen die Hydroxyprolin-Serumkonzentrationen deutlich an. Die Behandlung mit täglich 1 mg Dienogest über 14 Tage senkte statistisch signifikant die Hydroxyprolin-Konzentrationen auf das Niveau der scheinoperierten Tiere. Pro Versuchsgruppe wurden 8 Tiere eingesetzt. Histologisch wurde eine Hemmung der Osteoklasten durch Dienogest belegt. (Siehe Abbildung)

### Beispiel 5

### Dienogest-Salbe

| | | | | |
|---|---|---|---|---|
| Arzneistoff | Dienogest 1 g Salbe enthält 0.090 mg Dienogest | | | |
| | | | | |
| Hilfsstoffe | Eudispert | 12.5 g (1) | Tween 80 | 5 g (6) |
| | VE-Wasser ^{(x)} | 125 g (2) | Miglyol | 250 g (7) |
| | Glycerol 100 % | 50 g (3) | | |
| | NaOH | 3 g (4) | | |
| | VE-Wasser | 59,5 g (5) | | |

| | | | | |
|---|---|---|---|---|
| Herstellung: (1) wird in dem Gemisch von (2) und (3) quellen gelassen, auf 45 °C erwärmt. Hierzu wird nach Anquellung die durch Hinzufügen von (4) in (5) erhaltene Lösung langsam hinzugegeben. Nach 30sekundiger Dispergierung bei 20 000 U/Min. wird (6) hinzugefügt und anschließend bei gleicher Dispergierung innerhalb von 20 Min. zuerst das Dienogest und anschließend (7) zugegeben und intensiv vermischt. ^{(x)} VE-Wasser = vollentsalztes Wasser | | | | |

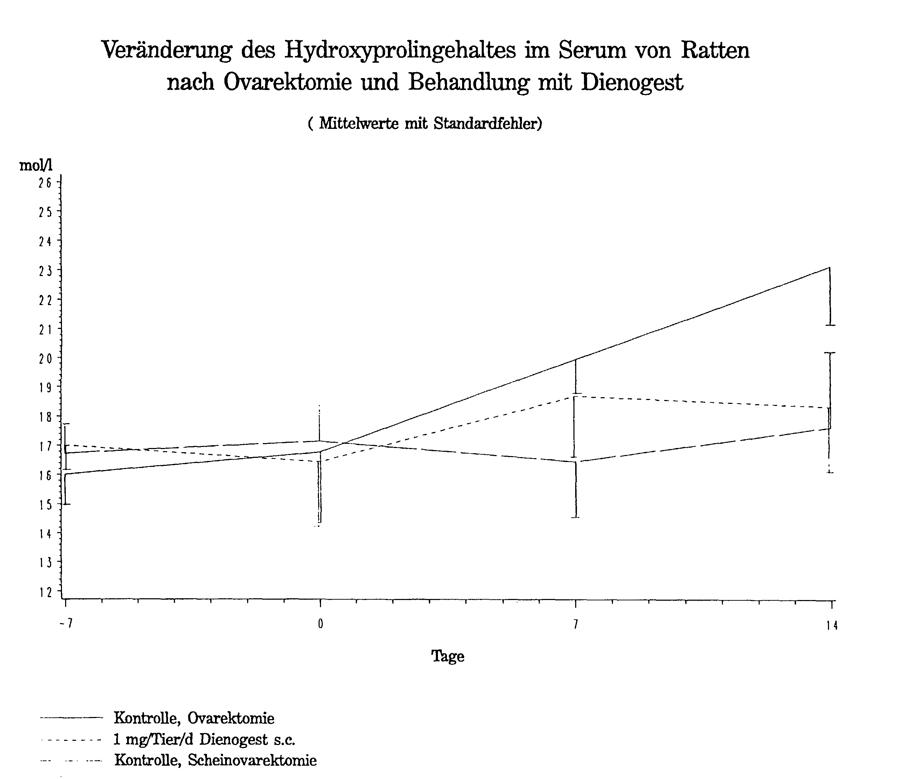

## Patentansprüche

1. Pharmazeutische Zubereitung auf Gestagen-Basis,
**dadurch gekennzeichnet,**
**daß** sie zu einem Anteil aus einem Carboxyvinylpolymeren
oder dessen Neutralisationsprodukt besteht, in das das Gestagen eingebettet ist
und die pharmazeutische Zubereitung aus Gelen und/oder sich im Kontakt mit dem Organismus in Gele umwandelnde überzogenen Arzneiformen, Salben, transdermalen therapeutischen Systemen oder anderen Zubereitungen besteht.

2. Pharmazeutische Zubereitung nach Anspruch 1,
**dadurch gekennzeichnet,**
**daß** der Anteil des Carboxyvinylpolymeren zwischen 0,5 und 50 % beträgt und daß das Carboxyvinylpolymere aus Copolymerisaten aus einem oder mehreren Acryl- und/ oder Methacrylestern mit Acryl-, Methacryl-, Itacon-, Croton-, Malein- oder Fumarsäure besteht.

3. Pharmazeutische Zubereitung nach den Ansprüchen 1 bis 2,
**dadurch gekennzeichnet,**
**daß** die Copolymerisate einen Säureanteil zwischen 5 und 25 % aufweisen.

4. Pharmazeutische Zubereitung nach den Ansprüchen 1 bis 3,
**dadurch gekennzeichnet,**
**daß** die Neutralisationsprodukte der Carboxyvinylpolymere mit Natronlauge, mit Kalilauge,Stickstoffbasen oder anderen basischen Substanzen ganz oder teilweise neutralisierte Carboxyvinylpolymere darstellen.

5. Pharmazeutische Zubereitung nach den Ansprüchen 1 bis 4,
**dadurch gekennzeichnet,**
**daß** das Gestagen ein Steroid aus der Gruppe Dienogest, Chlormadinonacetat, Norgestimat, Lynestrenol, Levonorgestrel, Cyproteronacetat, Desogestrel, Gestoden sowie Norethisteronacetat darstellt.

6. Pharmazeutische Zubereitung nach den Ansprüchen 1 bis 5,
**dadurch gekennzeichnet,**
**daß** das Gel zuerst als Tablette, Dragee oder Kapsel vorliegt, die sich im Organismus im Ergebnis des Zerfalls in ein Gel umwandelt.

## Claims

1. Gestagen-based pharmaceutical preparation, **characterized in that** it consists partly of a carboxyvinyl polymer or its neutralization product, in which the gestagen is embodied, and the pharmaceutical preparation consists of gels and/or coated pharmaceutical forms converted into gels on contact with the body, ointments, transdermal therapeutic systems or other preparations.

2. Pharmaceutical preparation according to Claim 1, **characterized in that** the proportion of the carboxyvinyl polymer is between 0.5 and 50%, and that the carboxyvinyl polymer consists of copolymers of one or more acrylic and/or methacrylic esters with acrylic, methacrylic, itaconic, crotonic, maleic or fumaric acid.

3. Pharmaceutical preparation according to Claims 1 to 2, **characterized in that** the copolymers have an acid content between 5 and 25%.

4. Pharmaceutical preparation according to Claims 1 to 3, **characterized in that** the neutralization products of the carboxyvinyl polymers represent carboxyvinyl polymers wholly or partly neutralized with sodium hydroxide solution, or with potassium hydroxide solution, nitrogen bases or other basic substances.

5. Pharmaceutical preparation according to Claims 1 to 4, **characterized in that** the gestagen represents a steroid from the group of dienogest, chlormadinone acetate, norgestimate, lynestrenol, levonorgestrel, cyproterone acetate, desogestrel, gestodene and norethisterone acetate.

6. Pharmaceutical preparation according to Claims 1 to 5, **characterized in that** the gel is initially in the form of a tablet, a coated tablet or capsule which is converted in the body into a gel as the result of disintegration.

## Revendications

1. Préparation pharmaceutique à base d'un progestatif, **caractérisée en ce qu'**elle consiste pour une partie en un polymère carboxyvinylique ou en son produit de neutralisation, dans lequel est incorporé le progestatif, et la préparation pharmaceutique est constituée de gels et/ou de formes médicamenteuses enduites se transformant en gels au contact avec l'organisme, de pommades, de systèmes thérapeutiques transdermiques ou d'autres préparations.

2. Préparation pharmaceutique selon la revendication 1, **caractérisée en ce que** la proportion du polymère carboxyvinylique est comprise entre 0,5 et 50 % et **en ce que** le polymère carboxyvinylique consiste en copolymères d'un ou plusieurs esters acryliques et/ou méthacryliques et d'acide acrylique, méthacrylique, itaconique, crotonique, maléique ou fumarique.

3. Préparation pharmaceutique selon les revendications 1 et 2, **caractérisée en ce que** les copolymères présentent une teneur en acide comprise entre 5 et 25 %.

4. Préparation pharmaceutique selon les revendications 1 à 3, **caractérisée en ce que** les produits de neutralisation des polymères carboxyvinyliques représentent des polymères carboxyvinyliques partiellement ou totalement neutralisés avec une solution d'hydroxyde de sodium, avec une solution d'hydroxyde de potassium, des bases azotés ou d'autres substances basiques.

5. Préparation pharmaceutique selon les revendications 1 à 4, **caractérisée en ce que** le progestatif représente un stéroïde choisi parmi le diénogest, le chlormadinone acétate, le norgestimate, le lynestrénol, le lévonorgestrel, le cyprotérone acétate, le désogestrel, le gestodène ainsi que le noréthistérone acétate.

6. Préparation pharmaceutique selon les revendications 1 à 5 **caractérisée en ce que** le gel se trouve sous forme de comprimé, dragée ou gélule, qui se transforme en un gel dans l'organisme par suite de la désintégration.
